# EUROPEAN PATENT APPLICATION

(11) **EP 1 666 075 A1**
(43) Date of publication of application: **07.06.2006**
(21) Application number: 04023609.3
(22) Date of filing: 04.10.2004
(51) Int. Cl.: A61L 26/00, A61L 15/28

(54) **Wound dressing compositions, especially for delivery of protease inhibitors**

(71) Applicant: Switch Biotech Aktiengesellschaft, 82061 Neuried (DE); Ludwig-Maximilians-Universität München, 80359 München (DE)
(72) Inventor: Schmidt, Roland, 91801 Markt Berolzheim (DE); Winter, Gerhard, 82377 Penzberg (DE)
(74) Representative: Bösl, Raphael Konrad

(57) **Abstract**

The present invention relates to dry and hydrated, i.e. wet wound dressings and delivery systems also suitable for active ingredients, their use for the treatment of wounds and skin diseases, preferably chronic wounds, and methods of preparing them.

Wound dressings according to the invention comprise a cellulose ether, preferably hydroxyethyl cellulose, and a gellan gum and optionally a protease inhibitor such as alpha-1-antichymotrypsin.

SEQUENCE LISTING

<110> SWITCH Biotech Aktiengesellschaft; Ludwig-Maximilians-Universität
München

<120> Wound dressing compositions, especially for delivery of protease in-
hibitors.

<130> PAT35EP

<160> 5

<170> PatentIn version 3.2

<210> 1
<211> 423
<212> PRT
<213> Homo sapiens

<400> 1

<210>  2
<211>  400
<212>  PRT
<213>  Homo sapiens

<400>  2

His Pro Asn Ser Pro Leu Asp Glu Glu Asn Leu Thr Gln Glu Asn Gln
1               5                   10                  15

Asp Arg Gly Thr His Val Asp Leu Gly Leu Ala Ser Ala Asn Val Asp
            20                  25                  30

Phe Ala Phe Ser Leu Tyr Lys Gln Leu Val Leu Lys Ala Pro Asp Lys
        35                  40                  45

Asn Val Ile Phe Ser Pro Leu Ser Ile Ser Thr Ala Leu Ala Phe Leu
    50                  55                  60

Ser Leu Gly Ala His Asn Thr Thr Leu Thr Glu Ile Leu Lys Gly Leu
65                  70                  75                  80

Lys Phe Asn Leu Thr Glu Thr Ser Glu Ala Glu Ile His Gln Ser Phe
                85                  90                  95

Gln His Leu Leu Arg Thr Leu Asn Gln Ser Ser Asp Glu Leu Gln Leu
            100                 105                 110

Ser Met Gly Asn Ala Met Phe Val Lys Glu Gln Leu Ser Leu Leu Asp
        115                 120                 125

Arg Phe Thr Glu Asp Ala Lys Arg Leu Tyr Gly Ser Glu Ala Phe Ala
    130                 135                 140

Thr Asp Phe Gln Asp Ser Ala Ala Ala Lys Lys Leu Ile Asn Asp Tyr
145                 150                 155                 160

Val Lys Asn Gly Thr Arg Gly Lys Ile Thr Asp Leu Ile Lys Asp Leu
                165                 170                 175

Asp Ser Gln Thr Met Met Val Leu Val Asn Tyr Ile Phe Phe Lys Ala
            180                 185                 190

Lys Trp Glu Met Pro Phe Asp Pro Gln Asp Thr His Gln Ser Arg Phe
        195                 200                 205

Tyr Leu Ser Lys Lys Lys Trp Val Met Val Pro Met Met Ser Leu His
    210                 215                 220

His Leu Thr Ile Pro Tyr Phe Arg Asp Glu Glu Leu Ser Cys Thr Val
225                 230                 235                 240

Val Glu Leu Lys Tyr Thr Gly Asn Ala Ser Ala Leu Phe Ile Leu Pro
                245                 250                 255

Asp Gln Asp Lys Met Glu Glu Val Glu Ala Met Leu Leu Pro Glu Thr
            260                 265                 270

Leu Lys Arg Trp Arg Asp Ser Leu Glu Phe Arg Glu Ile Gly Glu Leu
        275                 280                 285

Tyr Leu Pro Lys Phe Ser Ile Ser Arg Asp Tyr Asn Leu Asn Asp Ile
    290                 295                 300

Leu Leu Gln Leu Gly Ile Glu Glu Ala Phe Thr Ser Lys Ala Asp Leu
305                 310                 315                 320

Ser Gly Ile Thr Gly Ala Arg Asn Leu Ala Val Ser Gln Val Val His
                325                 330                 335

Lys Ala Val Leu Asp Val Phe Glu Glu Gly Thr Glu Ala Ser Ala Ala
            340                 345                 350

Thr Ala Val Lys Ile Thr Leu Leu Ser Ala Leu Val Glu Thr Arg Thr
        355                 360                 365

Ile Val Arg Phe Asn Arg Pro Phe Leu Met Ile Ile Val Pro Thr Asp
    370                 375                 380

Thr Gln Asn Ile Phe Phe Met Ser Lys Val Thr Asn Pro Lys Gln Ala
385                 390                 395                 400


<210>  3
<211>  398
<212>  PRT
<213>  Homo sapiens

<400>  3

Asn Ser Pro Leu Asp Glu Glu Asn Leu Thr Gln Glu Asn Gln Asp Arg
1               5                   10                  15

Gly Thr His Val Asp Leu Gly Leu Ala Ser Ala Asn Val Asp Phe Ala

20                  25                  30

Phe Ser Leu Tyr Lys Gln Leu Val Leu Lys Ala Pro Asp Lys Asn Val
        35                  40                  45

Ile Phe Ser Pro Leu Ser Ile Ser Thr Ala Leu Ala Phe Leu Ser Leu
    50                  55                  60

Gly Ala His Asn Thr Thr Leu Thr Glu Ile Leu Lys Gly Leu Lys Phe
65                  70                  75                  80

Asn Leu Thr Glu Thr Ser Glu Ala Glu Ile His Gln Ser Phe Gln His
                85                  90                  95

Leu Leu Arg Thr Leu Asn Gln Ser Ser Asp Glu Leu Gln Leu Ser Met
            100                 105                 110

Gly Asn Ala Met Phe Val Lys Glu Gln Leu Ser Leu Leu Asp Arg Phe
        115                 120                 125

Thr Glu Asp Ala Lys Arg Leu Tyr Gly Ser Glu Ala Phe Ala Thr Asp
    130                 135                 140

Phe Gln Asp Ser Ala Ala Ala Lys Lys Leu Ile Asn Asp Tyr Val Lys
145                 150                 155                 160

Asn Gly Thr Arg Gly Lys Ile Thr Asp Leu Ile Lys Asp Leu Asp Ser
                165                 170                 175

Gln Thr Met Met Val Leu Val Asn Tyr Ile Phe Phe Lys Ala Lys Trp
            180                 185                 190

Glu Met Pro Phe Asp Pro Gln Asp Thr His Gln Ser Arg Phe Tyr Leu
        195                 200                 205

Ser Lys Lys Lys Trp Val Met Val Pro Met Met Ser Leu His His Leu
    210                 215                 220

Thr Ile Pro Tyr Phe Arg Asp Glu Glu Leu Ser Cys Thr Val Val Glu
225                 230                 235                 240

Leu Lys Tyr Thr Gly Asn Ala Ser Ala Leu Phe Ile Leu Pro Asp Gln
                245                 250                 255

Asp Lys Met Glu Glu Val Glu Ala Met Leu Leu Pro Glu Thr Leu Lys
            260                 265                 270

Arg Trp Arg Asp Ser Leu Glu Phe Arg Glu Ile Gly Glu Leu Tyr Leu
        275                 280                 285

Pro Lys Phe Ser Ile Ser Arg Asp Tyr Asn Leu Asn Asp Ile Leu Leu
    290                 295                 300

Gln Leu Gly Ile Glu Glu Ala Phe Thr Ser Lys Ala Asp Leu Ser Gly
305                 310                 315                 320

Ile Thr Gly Ala Arg Asn Leu Ala Val Ser Gln Val Val His Lys Ala
                325                 330                 335

Val Leu Asp Val Phe Glu Glu Gly Thr Glu Ala Ser Ala Ala Thr Ala
            340                 345                 350

Val Lys Ile Thr Leu Leu Ser Ala Leu Val Glu Thr Arg Thr Ile Val
        355                 360                 365

Arg Phe Asn Arg Pro Phe Leu Met Ile Ile Val Pro Thr Asp Thr Gln
    370                 375                 380

Asn Ile Phe Phe Met Ser Lys Val Thr Asn Pro Lys Gln Ala
385                 390                 395

<210> 4
<211> 401
<212> PRT
<213> Artificial

<220>
<223> mature human ACT according to SEQ ID No. 2 having an additional
N-terminal methionine

<400> 4

Met His Pro Asn Ser Pro Leu Asp Glu Glu Asn Leu Thr Gln Glu Asn
1               5                   10                  15

Gln Asp Arg Gly Thr His Val Asp Leu Gly Leu Ala Ser Ala Asn Val
            20                  25                  30

Asp Phe Ala Phe Ser Leu Tyr Lys Gln Leu Val Leu Lys Ala Pro Asp
        35                  40                  45

Lys Asn Val Ile Phe Ser Pro Leu Ser Ile Ser Thr Ala Leu Ala Phe
    50                  55                  60

Leu Ser Leu Gly Ala His Asn Thr Thr Leu Thr Glu Ile Leu Lys Gly
65                  70                  75                  80

Leu Lys Phe Asn Leu Thr Glu Thr Ser Glu Ala Glu Ile His Gln Ser
                85                  90                  95

Phe Gln His Leu Leu Arg Thr Leu Asn Gln Ser Ser Asp Glu Leu Gln
            100                 105                 110

Leu Ser Met Gly Asn Ala Met Phe Val Lys Glu Gln Leu Ser Leu Leu
        115                 120                 125

Asp Arg Phe Thr Glu Asp Ala Lys Arg Leu Tyr Gly Ser Glu Ala Phe
    130                 135                 140

Ala Thr Asp Phe Gln Asp Ser Ala Ala Ala Lys Lys Leu Ile Asn Asp
145                 150                 155                 160

Tyr Val Lys Asn Gly Thr Arg Gly Lys Ile Thr Asp Leu Ile Lys Asp
                165                 170                 175

Leu Asp Ser Gln Thr Met Met Val Leu Val Asn Tyr Ile Phe Phe Lys
            180                 185                 190

Ala Lys Trp Glu Met Pro Phe Asp Pro Gln Asp Thr His Gln Ser Arg
        195                 200                 205

Phe Tyr Leu Ser Lys Lys Lys Trp Val Met Val Pro Met Met Ser Leu
    210                 215                 220

His His Leu Thr Ile Pro Tyr Phe Arg Asp Glu Glu Leu Ser Cys Thr
225                 230                 235                 240

Val Val Glu Leu Lys Tyr Thr Gly Asn Ala Ser Ala Leu Phe Ile Leu
                245                 250                 255

Pro Asp Gln Asp Lys Met Glu Glu Val Glu Ala Met Leu Leu Pro Glu
            260                 265                 270

Thr Leu Lys Arg Trp Arg Asp Ser Leu Glu Phe Arg Glu Ile Gly Glu
        275                 280                 285

Leu Tyr Leu Pro Lys Phe Ser Ile Ser Arg Asp Tyr Asn Leu Asn Asp
    290                 295                 300

Ile Leu Leu Gln Leu Gly Ile Glu Glu Ala Phe Thr Ser Lys Ala Asp
305                 310                 315                 320

Leu Ser Gly Ile Thr Gly Ala Arg Asn Leu Ala Val Ser Gln Val Val
                325                 330                 335

His Lys Ala Val Leu Asp Val Phe Glu Glu Gly Thr Glu Ala Ser Ala
            340                 345                 350

Ala Thr Ala Val Lys Ile Thr Leu Leu Ser Ala Leu Val Glu Thr Arg
        355                 360                 365

Thr Ile Val Arg Phe Asn Arg Pro Phe Leu Met Ile Ile Val Pro Thr
    370                 375                 380

Asp Thr Gln Asn Ile Phe Phe Met Ser Lys Val Thr Asn Pro Lys Gln
385                 390                 395                 400

Ala

<210>  5
<211>  399
<212>  PRT
<213>  Artificial

<220>
<223>  mature human ACT according SEQ ID No. 3 having an additional
       N-terminal Methionine

<400>  5

Met Asn Ser Pro Leu Asp Glu Glu Asn Leu Thr Gln Glu Asn Gln Asp
1               5                   10                  15

Arg Gly Thr His Val Asp Leu Gly Leu Ala Ser Ala Asn Val Asp Phe
            20                  25                  30

Ala Phe Ser Leu Tyr Lys Gln Leu Val Leu Lys Ala Pro Asp Lys Asn
        35                  40                  45

Val Ile Phe Ser Pro Leu Ser Ile Ser Thr Ala Leu Ala Phe Leu Ser
    50                  55                  60

Leu Gly Ala His Asn Thr Thr Leu Thr Glu Ile Leu Lys Gly Leu Lys
65                  70                  75                  80

Phe Asn Leu Thr Glu Thr Ser Glu Ala Glu Ile His Gln Ser Phe Gln
                85                  90                  95

His Leu Leu Arg Thr Leu Asn Gln Ser Ser Asp Glu Leu Gln Leu Ser
            100                 105                 110

Met Gly Asn Ala Met Phe Val Lys Glu Gln Leu Ser Leu Leu Asp Arg
        115                 120                 125

Phe Thr Glu Asp Ala Lys Arg Leu Tyr Gly Ser Glu Ala Phe Ala Thr
    130                 135                 140

Asp Phe Gln Asp Ser Ala Ala Ala Lys Lys Leu Ile Asn Asp Tyr Val
145                 150                 155                 160

Lys Asn Gly Thr Arg Gly Lys Ile Thr Asp Leu Ile Lys Asp Leu Asp
                165                 170                 175

Ser Gln Thr Met Met Val Leu Val Asn Tyr Ile Phe Phe Lys Ala Lys
            180                 185                 190

Trp Glu Met Pro Phe Asp Pro Gln Asp Thr His Gln Ser Arg Phe Tyr
        195                 200                 205

Leu Ser Lys Lys Lys Trp Val Met Val Pro Met Met Ser Leu His His
    210                 215                 220

Leu Thr Ile Pro Tyr Phe Arg Asp Glu Glu Leu Ser Cys Thr Val Val
225                 230                 235                 240

Glu Leu Lys Tyr Thr Gly Asn Ala Ser Ala Leu Phe Ile Leu Pro Asp
                245                 250                 255

Gln Asp Lys Met Glu Glu Val Glu Ala Met Leu Leu Pro Glu Thr Leu
            260                 265                 270

Lys Arg Trp Arg Asp Ser Leu Glu Phe Arg Glu Ile Gly Glu Leu Tyr
        275                 280                 285

Leu Pro Lys Phe Ser Ile Ser Arg Asp Tyr Asn Leu Asn Asp Ile Leu
    290                 295                 300

Leu Gln Leu Gly Ile Glu Glu Ala Phe Thr Ser Lys Ala Asp Leu Ser
305                 310                 315                 320

Gly Ile Thr Gly Ala Arg Asn Leu Ala Val Ser Gln Val Val His Lys
                325                 330                 335

Ala Val Leu Asp Val Phe Glu Glu Gly Thr Glu Ala Ser Ala Ala Thr
            340                 345                 350

Ala Val Lys Ile Thr Leu Leu Ser Ala Leu Val Glu Thr Arg Thr Ile
        355                 360                 365

Val Arg Phe Asn Arg Pro Phe Leu Met Ile Ile Val Pro Thr Asp Thr

    370                 375                 380

Gln Asn Ile Phe Phe Met Ser Lys Val Thr Asn Pro Lys Gln Ala
385                 390                 395

## Description

The present invention relates to dry and hydrated, i.e. wet wound dressings and delivery systems also suitable for active ingredients, their use for the treatment of wounds and skin diseases, preferably chronic wounds, and methods of preparing them. More particularly, the present invention relates to a xerogel or film comprising a mixture of a cellulose ether and a gellan gum. Such systems can be used directly as wound dressings or alternatively as dry, storage stable delivery systems for active ingredients, preferably proteins, in the field of cosmetics and medicine. Before use and/or during application in a moist environment, especially wounds, the dry composition is rehydrated, thus serving as a hydrogel loaded with active ingredients which are released at a controlled rate. Depending on the ionic strength of the hydrating solution the resulting gel can be immobilised and thus the composition can not flow out of the wound e.g. under the influence of gravity. Such a system can be used for moist wound healing or dermal delivery of therapeutic substances and for other medical or cosmetic purposes.

In an especially preferred embodiment, the wound dressing according to the invention comprises a cellulose ether, a gellan gum and alphal-antichymotrypsin (ACT). Such compositions are characterized by excellent release characteristics for ACT and exceptional storage stability regarding ACT when stored in dry from.

### Background of the invention.

The present invention can be used for delivery of active ingredients especially alpha1-antichymotrypsin into wounds. The growing number of patients with diabetes mellitus, venous insufficiency and other chronic diseases and injuries have resulted in an increased incidence of chronic non-healing soft-tissue wounds of the skin: Apart from causing great costs to the public health system, chronic wounds give raise to a very painful, distressing condition of the patients and may even lead to amputation. Therefore, adequate treatment and promotion of dermal wound healing is necessary.

The mechanisms of wound healing in general and characteristics of different wound healing phases are well known. Since 1962 moist wound healing has become a widely accepted treatment. Already many different moist bandages, like hydrocolloid, hydropolymer or alginate systems are on the market. These bandages shall ensure a moist environment in the wound. Sometimes they additionally take up wound secretions while they swell or in exchange with solutions, which are incorporated into the bandages (for example ringer solution). All these wound dressings consist of a swollen or swellable polymer and sometimes of a water resistant backing layer, but they usually do not contain any therapeutic substances.

Next to a moist environment, adequate concentrations of growth factors are necessary to promote healing. In the 1970s the inductive effect of platelet derived factors and other cytokines was first described. The necessary balance and concentration of different growth factors for the promotion of healing is often disturbed, especially in elderly people and/or patients with diabetes or autoimmune disorders. Therefore, it was proposed to apply wound healing factors like PDGF, TGF-β, F XIII, KGF- or EGF to enumerate just few, topically into wounds. Moreover, protease inhibitors have been proposed to promote healing of chronic wounds. Especially, alphal-antichymotrypsin has been described as being useful in stimulating the wound healing process, particularly in diabetic wounds (WO 02/088180).

A problem which has often been encountered is that the topical delivery system for active ingredient does not adequately stabilize or release the active ingredient and/or that it is not adequate regarding patient compliance. Especially for proteins as active ingredients, which are typically labile, the above prerequisites are difficult to meet.

Drug solutions with low viscosity evaporate fast and therefore have to be applied very often. Although solutions are easy to apply the persistent contact with the wound surface is not good because a solution does not adhere to the site of action on the wound surface. Moreover, growth factors, enzymes, and some inhibitors which promote wound healing in the first inflammatory phase are proteins and therefore quite unstable and sensitive molecules. When stored in aqueous media at room temperature many proteins do not remain stable, they may aggregate and lose activity rapidly. EGF in an aqueous formulation for example lost 40% of its activity within two weeks (Clanan,D.P. *et al* Potency and stability of C terminal truncated human epidermal growth factor. *Gut* 47, 622-627 (2000)). To achieve longer storage stabilities protein solutions have to be stored under defined conditions at deep temperatures (-20°C or 4-8°C). Therefore, these aqueous products have technical, economical and handling drawbacks. E.g. the solution Eurokinin, a cleaned solution of the patients own growth factors, must be stored in a freezer. This leads to additional problems as it has to be carefully thawed before application which decreases patient and medical personnel compliance. An alternative to deep temperature storage is the stabilization of a sensitive substance in dry products. It is a widespread method in pharmaceutical technology to embed sensitive substances like proteins in dry, amorphous matrices to ensure low degradation rates. Thus, there is a high need for the development of dry, storage stable active ingredient products. It is often not possible to design formulations based merely on the lyophilization of the bulk solutions, as the amounts of active ingredient used in the formulation for a single dose will usually be very small and as during the lyophilization process the drug may accidentally be pulled from the lyophilization container by the vacuum employed in the process. Furthermore many proteins are relatively unstable when lyophilised in small concentrations. They can adsorb to product packaging and lose activity. This may only be overcome by the use of a diluent or extender to increase the amount of solid present during the process.

Moreover, hydrogels are preferred over solutions with low viscosity as they keep the wound moist, do not evaporate fast and therefore have to be applied only once daily. The solution Eurokinin for example has to be applied continuously onto a compress on the wound. Regranex is a hydrogel, which shows good wound healing and handling properties, but inadequate storage stabilities. Gels can provide a controlled delivery system for protein on a wound site. Controlled release refers to a drug release sufficient to maintain a therapeutic level over an extended period of time. This is an important advantage because it permits less frequent application of the formulation to the wound and thereby permits less disturbance of the wound. A variety of gels and ointments for application to wounds are described in the prior art for a variety of purposes. They may for example be used to clean wounds, to promote healing of the wound or to prevent infection. In certain circumstances, the gel may include an active ingredient which is administered to the patient by topical application of the gel. One example of a commercially available wound gel is Intrasite® produced by Smith & Nephew ltd. This gel contains hydrated carboxymethyl cellulose-Na as a main ingredient and is packaged and applied to wounds in gel form as a primary treatment in order to debride the wound.

Gels have the further advantage of having a high water content, easy application to a wound, and easy removal from the wound after application by washing. The gel may also assist in preventing the wound from drying out, thereby promoting the healing process. Since gels are mobile they offer the advantage of intimate contact with the often irregular surface of a wound, something that is often not achieved with a more rigid or liquid wound dressing. The advantage of good contact is however tempered by the conflicting needs of making the gel sufficiently mobile that it can be applied to the wound but not so mobile that it runs out of the wound under the influence of gravity. Gels currently in use suffer from the disadvantage that they can run out of the wound.

However, other problems of existing topical products have to be faced as for example the absence of exact and reproducible dosage. All hydrogels on the market like Regranex® are dosed by the amount of the applied hydrogel strand which is not sufficiently exact and reproducible.

An alternative to deep temperature storage of wet drug forms is the stabilization of a sensitive substance in dry products. It is a widespread method in pharmaceutical technology to embed sensitive substances like proteins in dry, amorphous matrices to ensure low degradation rates. Thus, there is a high need for the development of dry, storage stable active ingredient products. These dry products have to be rehydrated before or during use, as for wound healing the application of moist products, like preferably hydrogels, is necessary.

The drying procedure can be carried out by various methods. Freeze drying and warm air drying are preferred drying procedures for the present invention. Particularly preferred is freeze-drying. Drying methods using organic solvents are less preferred due to the unwanted interactions of labile active ingredients with organic solvents and the problems of residual solvent contents in the product that at least affords additional testing routines adding to production costs.

A dry storage system can either be hydrated before use with a suitable aqueous solution and/or during use in contact with aqueous body fluids, especially when applied topically to a wound. Preferred dry storage systems for hydrogels are films and xerogels, especially xerogels. The xerogel or film take up water upon hydration, swell and eventually form a hydrogel.

A desireable, optimal active ingredient product would have a dry, storage stable form and could be hydrated to a hydrogel before use. Such storage stable forms need not to be stored at very low temperatures and thus allow also easy and cheap transportation. Moreover, such products may also be stored by the patient himself without complications. Thereby, high costs which occur when treatment has to be effected at hospitals are avoided. It would be useful to have a dry, ready to use, single dose product covering a defined contact area, which can also be cut reproducibly into pieces in order to achieve defined doses. Moreover, it would be a great advantage if the patient to be treated could adapt the medicament or cosmetic composition according to his needs, for example to immobilize the gel by hydration to prevent flowing out of the wound or to reconstitute it to a mobile gel that can insert in even cleft regions of a wound without gaps.

It is described in the prior art to mix an active medicament unstable to heat with a biodegradable protein carrier such as collagen, atelocollagen, or gelatin to form a carrier matrix having sustained release properties. The resultant mixture is then dried, and the dried material is formed into an appropriate shape, as described in US 4,774,091. Examples of active ingredients for this purpose are given as t-Pa, prostaglandines, prostacyclines biohormones e.g. hGH, bGH, GRF, somatomedins, and calcitonin, interferons, interleukins, tumor necrosis factor and other cytokines such as macrophage activating factor, migration inhibitory factor, and colony stimulating factor. JP 2002143290 describes a freeze dried matrix made from a gel formed by a mixture of PLGA-copolymer and bovine atelocollagen I. Additionally, the matrix is cross-linked by glutaraldehyde vapour. EP 083491A2 discloses a polysaccharide sponge made by solvent drying. US 5,189,148 describes a stabilized FGF composition and production thereof. US 2003/0105007 discloses a solid formulation for growth factors, e.g. PDGF in fibronectin. WO 91/19480 describes a freeze-dried hydrogel preparation containing a wound healing medicament which is compressed after freeze drying. The polymer in this invention is Hydroxyethylcellulose (HEC). EP 0308238A1 describes stable lyophilised formulations containing growth factors. US 5,714,458 discloses a stable lyophilised formulation of FGF in a xerogel. DE 19503338 A1 describes a film for the release of collagenase. The film can consist of various polymers. Gellan gum is not mentioned. WO03/034993 and GB 1102118 describe collagen sponges as wound/tissue healing material. CA2246895 describe bioabsorbable solid materials including films and sponges.

Cellulose derivatives have been used to formulate therapeutic proteins or polypeptides for topical use. See, e.g. EP 267,015, EP 308,238, and EP 312 208, which disclose formulations of a polypeptide growth factor having mitogenic activity, such as TGF beta, in a polysaccharide such as methylcellulose. EP 261,599 discloses human topical applications such as TGF beta. EP 193,917 discloses a slow release composition of a carbohydrate polymer such as cellulose and a protein such as growth factor. GB 2,160,528 describes a formulation of a bioactive protein and a polysaccharide. US 4,609,640 describes a therapeutic agent and a polymer selected from polysaccharides, cellulose, starches, dextroses. DE4328329 claims a freeze dried biomatrix consisting of natural polysaccharides and modified polysaccharides. GB 2357765 describes an alginate foam crosslinked by di- and trivalent cations.

The above-described wound dressing materials provide important advantages. The materials are of natural, biological origin (albeit chemically modified), and consequently tend to have low antigenicity. Furthermore, some of these materials can have positive therapeutic effects on wound healing. In principle, they are suitable for the delivery of protein active ingredients.

However, some difficulties remain. For example, a drawback of some collagen- and gelatine-based wound dressing materials is that the collagen breaks down too fast in vivo, due to the action of collagenase enzymes in the wound. This can be countered to some extent by cross-linking the collagen/gelatine with a covalent cross-linking agent such as glutaraldehyde or dicyclohexylcarbodiimide. Residual contents of these agents, however, can cause unwanted interactions with loaded protein active ingredients and the wound milieu itself. Besides, by cross-linking a strong irreversible immobilization is effected. So depending on the type of wounds, the immobile sponge in the first period after application may not cover cleft wound grounds and in the later stages the sponge is liquefied by enzymatic degradation what as well does not provide good contact to the wound ground. Moreover, collagen as a component of wound dressings is prone to denaturation when it is sterilized by gamma-irradiation. Collagen and gelatine also are extracted from natural animal sources. So, the problems of contamination with pathogens for animal or human, like the bovine spongiform encephalopathy (BSE) and others are relevant. Moreover, it can be antigenic to certain patients unless stringent measures are taken to purify the collagen, which add to its cost.

Non cross-linkable materials like non-ionic cellulose ethers suffer from the rheological disadvantage that they can run out of the wound and so lose close contact with the wound surface. Materials that are covalently cross-linked are always endangered of having unwanted effects caused by residual contents of cross-linking agents antagonising wound healing. Di- and trivalent cations used e.g. for crosslinking alginate also can have antagonising effects on the wound healing process. Moreover, solutions of these cations in the needed high concentrations are not used in clinical practice. This complicates the possibility that the cross-linking is carried out by the applicant when it seems appropriate for the individual wound condition. Additionally, alginates are not suitable for steam sterilization.

Biodegradable polymers like PLGA, collagen, gelatine, and others in some cases are known to interact with labile drugs due to their fragmentation. So, fragments of PLGA, i.e. lactic acid and glycolic acid can change pH and the osmotic pressure within the matrix to awkward proportions. The manufacture of matrices consisting of polymers unsuitable for steam sterilization, e.g. hydroxypropyl cellulose, hydroxypropylmethyl cellulose, collagen, and gelatine, need a more complicated and expensive sterilization process, e.g. gamma irradiation, which adds to its costs. Sodium salts of polymers like carboxymethyl cellulose Sodium or carboxymethyl starch Sodium are unfavourable for lyophilisation because their combination with phosphate buffer, which is essential for many protein formulations, results in a pH-shift into the sour region which is counterproductive for protein stability.

Therefore, there remains a need for drug delivery compositions for topical use, especially improved wound dressing materials exhibiting control of physical properties, therapeutic effects on wound healing, reduced costs, and material safety in terms of reduced antigenic response and circumvention of unwanted effects of fragments or counterions of matrix molecules.

It is an object of the present invention to provide improved drug delivery compositions for topical use, especially wound dressing materials suitable for delivery of active ingredients if necessary, to mammalian wounds, and especially to human chronic wounds, such as venous ulcers, decubitus ulcers and diabetic ulcers. We make a new combination of dressing materials available, which exhibit a mechanical i.e. rheological behaviour which can be controlled and adapted easily by the patient simply by choosing the hydration medium. Moreover, the wound dressing compositions of the invention are surprisingly suitable for stabilizing and releasing proteins, in particular alpha1-antichymotrypsin.

The present invention relates a wound dressing composition comprising a cellulose ether and a gellan gum.

In a preferred embodiment the cellulose ether is hydroxyethyl cellulose (HEC).

In another preferred embodiment the gellan gum is a deacetylated gellan gum.

In a even more preferred embodiment, the gellan gum is available under the trade name Kelcogel F.

In an especially preferred embodiment, the composition comprises hydroxyethyl cellulose (HEC) and deacetylated gellan gum.

In a preferred embodiment, the cellulose ether and the gellan gum are homogeneously mixed.

In a preferred embodiment the composition is in dry form, particularly a xerogel or film.

In a preferred embodiment the wound healing composition is a hydrogel. In another embodiment the hydrogel is obtained by hydration of a dry storage form of the gel, in particular by hydration of a xerogel or film.

The composition may contain, if desired, one or more additional excipients like sugars, sugar alcohols, surfactants, amino acids, antioxidants, polyethylene glycols. In a preferred embodiment, the excipients comprise at least one non-ionic surface active component preferably selected from Poloxamer® 188, Tween 80 and Tween 20.

In a preferred embodiment, the excipients comprise at least one buffer agent, preferably selected from phosphate and Tris.

In a preferred embodiment, the excipients comprise at least one amino acid, preferably arginine.

In a preferred embodiment, the excipients comprise at least one polyethylene glycol, preferably selected from PEG 400 suitable as plastiziser and PEG 2000 suitable as pore former.

In a preferred embodiment, the excipients comprise at least one polyvinyl pyrrolidone, preferably Kollidon 17PF, qhich is suitable as strengthener or pore former or stabilizer.
The wound dressing compositions according to the present invention preferably are a homogeneous mixture of gellan gum and hydroxyethyl cellulose.

In a preferred embodiment, the wound dressing composition comprising the homogeneous mixture of gellan gum and hydroxyethyl cellulose is in a suitable vehicle, such as a solvent, in a even more preferred embodiment the solvent is aqueous and the composition is a hydrogel. In another embodiment the hydrogel is formed by hydration of a film, xerogel or sponge.

In another preferred embodiment, the the wound dressing composition comprising the homogeneous mixture of gellan gum and hydroxyethyl cellulose is dry. In an even more preferred embodiment the dry composition is a film or a sponge or xerogel.

The mixture of the two components the gellan gum and the hydroxyethyl cellulose synergistically combine their benefits and complement one another to prevent their disadvantages observed when used alone.

Hydroxyethyl cellulose (HEC) can be obtained by the process described in US 4,084,060. HEC is a non-ionic water-soluble cellulose ether, formed by reaction of cellulose with ethylene oxide. It is widely used in pharmaceutical compositions and very well known in the art. It can be dispersed in cold or hot liquids, but is insoluble in most organic solvents. This material offers numerous advantages including the features that it is biocompatible, biostable, non-immunogenic and readily commercially available. It can be obtained in various degrees of molecular weight at high levels of purity. Preferably, the hydroxyethyl cellulose has a molecular weight of at least 500 kDa. Gels of hydroxyethyl cellulose can be steam sterilized.

Processes for producing gellan gum are well-known in the art, e.g., in US 4,326,052, US 4,326,053, US 4,377,636, US 4,385,126, and US 4,503,084. Particularly preferred is deacylated gellan gum, which is for example described in US 4,326,052. Gellan gum is a microbial polysaccharide derived from Pseudomonas elodea. It consists of a tetrasaccharide unit, □-D-glucose, □-D-glucuronic acid, □-D-glucose and □-L-rhamnose. (→ 3)-b-D-Glcp-(1→ 4)-b-D-GlcpA-(1→ 4)-b-D-Glcp-(1→ 4)-a-L-Rhap-(1→). Gellan is produced with two acyl substituents present on the 3-linked glucose, namely, L-glyceryl, positioned at O(2) and an acetyl substituent at 0(6). The native polysaccharide is partially esterified; the 1,3-D-Glc residue can be linked to L-glycerate at C-2 and/or to acetate at C-6, and there is 1 mol of glycerate per repeating unit and 0.5 mol of acetate per repeating unit. Acyl substituents affect the rheology of gels, and deacylation of native gellan results in a change from soft, elastic, thermoreversible gels to harder, more brittle gels. Gellan gum has been suggested for use in wound healing as solution/gel in US 6,596,704 and fibers in EP 0454373 as well as in WO 95/05204. Gellan gum has the CAS No. 71010-52-1. Well known Gellan gum products are available under the tradenames Gelrite® and Kelcogel®. "deacetylated Gellan Gum" according to the present invention is to be understood as gellan Gum obtainable by partial or complete deacetylation of native Gellan Gum. Preferably, the deacetylated Gellan Gum contains less than 10%, more preferably less than 25%, in particular less than 50%, most preferably less than 75% of the acetyl groups present in native Gellan Gum. Native Gellan Gum can be obtained by aerobic fermentation using *Sphingomonas elodea.*

Deacylated Gellan gum is readily soluble in water and is characterized by high viscosity at low concentration. The gum also has a high rheological yield point. Changes of pH in the 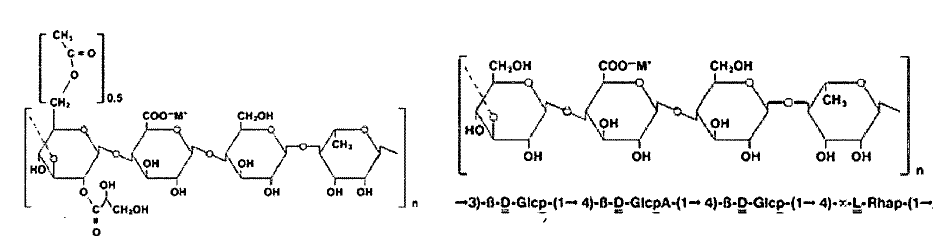 range 3-11 do not substantially affect the viscosity of the gel. The viscosity of such gel is also stable in the range of 20°C - 70°C. Above this temperature it reversibly liquefies; i.e. the thermoreversibility is formed after heating and cooling. Following heating in the presence of various cations deacetylated gellan gum produces firm, non-elastic or brittle gels. Cations especially useful in the formation of gels with are those of sodium, potassium, magnesium, calcium. Gellan gum is used as a thickening, suspending and stabilizing agent in aqueous systems in various fields including nutrition.

The common advantages of gellan gum and hydroxyethyl cellulose are that both components can promote wound healing. Moreover both gelling agents can be sterilized by steam sterilization, which strongly simplifies production processes. The swelling and thermoreversible behaviour of gellan gum also are activated by steam sterilization. Moreover, they can be lyophilised and they also form stable films when air dried. Also, a very low content of each gelling agent is needed for forming gels with high viscosities. As a result of the low concentrations of both HEC and gellan gum needed for the manufacture of a hydrogel of the invention, the pore sizes of freeze dried hydrogels of the invention (i.e. xerogel) are very high and the thickness of dired hydrogels of the invention (i.e. films) are very low, respectively. Together with the high hydrophilicity of both HEC and gellan gum, dry wound healing compositions of the invention show a strong and fast hydration and swelling which make them suitable for application in wound care. Moreover, both HEC and gellan gum are commercially available with a range of defined and controllable properties offering the possibility to adjust and control the properties of the wound healing compositions of the present invention as needed to an exceptional degree. In particular, the mechanical texture, viscosity, the rate of hydration, porosity and density of the materials can be adjusted in a wide range as it seems appropriate.

However, hydroxyethyl cellulose has the disadvantage that it is a non-crosslinked gelling agent. Such HEC-gels not containing gellan gum show a pseudoplastic rheology. That means there is no rheologic yield point and it is therefore free-flowing under the influence of gravity. The free-flowing HEC gels without gellan gum will flow out of the wound site and so lose contact with the wound ground resulting in a loss of their therapeutic effects.

Moreover, deacetylated gellan gum in the absence of HEC has the disadvantage that it forms brittle gels in presence of cations that would not be suitable for administration to a highly sensitive region such as a wound. Moreover, it lacks of the flexibility and flow tendency to be able to be applied without gaps to uneven and cleft wound surfaces. This however is essential for a wound dressing because the suitability of such wound dressing is greatly dependent on the interaction between the hydrogel wound dressing and the wound fluid: the release from the wound dressing can only take place by diffusion of the active substances at the interface between hydrogel wound dressing and wound fluid. For accurate, controlled, and reproducible release of drugs, especially in case of highly potent substances at low doses, e.g. proteins, a close contact between wound dressing and the wound is a prerequisite.

The mixture of a HEC and a gellan gum has all the advantages of the single components alone and overcomes disadvantages of the single components which makes the mixture exceptionally suitable for its use as wound healing composition. First, the rheology of the wound healing composition comprising a mixture of a gellan gum and HEC in the gel state can be adjusted as needed offering a broad range of advantageous wound dressing compositions:

Hydration of a xerogel/film comprising Gellan gum and HEC, and optionally at least one therapeutic wound healing agent with water:

The resulting gel behaves more like a HEC gel than a gellan gum gel. It still has a good flowability and is very smooth. The low salt contents incorporated into the matrix for other purposes, e.g. production of raw materials, pH adjustment, buffering, etc., provide only a very lowly pronounced yield point and plastic rheology. It is superior to a pure HEC gel in terms of running out of the wound site, but has the necessary flowability to creep into clefts of wounds.

Hydration of a xerogel/film comprising Gellan gum and HEC, and optionally at least one therapeutic wound healing agent with electrolyte solutions, e.g. isotonic NaCl, Ringer, etc:

The resulting gel is immobilized and provides a higher yield point and a highly pronounced plastic rheology. Moreover, it still is flexible enough to adjust to any surface morphology. Moreover, the soft pressure of a bandage is enough for the gel pad to overcome the yield point and start to creep and to deform to the given shape of the wound site.

Split up of the films/xerogels comprising Gellan gum and HEC, and optionally at least one therapeutic wound healing agent to fill extremely deep wounds:

For some very deep wound caves the flowability of the immobilized gel could not be sufficient. In that case the film/xerogel can be cut into parts. The part(s) for the deep cave of the wound ground can be hydrated in water. They will start to flow and creep into the deepest wholes of the wound like a normal free flowing hydrogel. The second part(s) will be hydrated in electrolyte solutions and get immobilized. They are placed over the first non-immobilized part(s) of the gel into the wound and serve as a clot. So the wound is completely filled without gaps, but the gel is still immobile and will stay in the wound accurately as long as desired.

Preferably, the weight ratio of HEC to gellan gum is in the range from 1:5 to 50:1, preferably from 1:1 to 10:1 1 in the wound dressing compositions of the invention .

Preferably, the hydroxyethyl cellulose and gellan gum together make up at least 25% by weight of the material on a dry weight basis, more preferably at least 30% by weight of the material on a dry weight basis, even more preferably at least 40% by weight of the material on a dry weight basis, particularly at least 50% by weight of the material on a dry weight basis. In wet hydrogels the content of gelling agent mixture; i.e the sum of HEC and gellan gum is preferably between 1 % and 10 %, preferably between 2 and 4%.

In one embodiment, the material essentially consists of the hydroxyethyl cellulose and gellan gum. In a preferred embodiment the composition is in form of a hydrogel or a dried or lyophilised form thereof, i.e. a film, xerogel or sponge. Such compositions may be used directly for topical applications in the field of cosmetics or medicine.

The composition according to the present invention may be in any convenient form, such as powder, microspheres, flakes, a mat, a sponge, a xerogel or a film.

In certain embodiments, the composition according to the present invention is in the form of a semisolid or gel ointment for topical application. In a preferred embodiment the wound dressing compositions of the present invention are in form of a hydrogel.

In another preferred embodiment, the wound dressing compositions of the present invention are in form of a freeze-dried or solvent-dried xerogel or sponge.

In yet other embodiments, the composition according to the present invention is in the form of a flexible film, which may be continuous or interrupted (e.g. perforated).

In a preferred embodiment, the flexible film preferably comprises a plasticiser to render it flexible, such as PEG 400, glycerol, or propylenglycol.

In another embodiment the invention relates to a wound dressing package comprising a sterile wound dressing composition of the invention, packaged in a microorganism-impermeable container. Such packages may usually represent single or multi unit dosage forms of the present compositions. These packages can be easily used by the patient himself, especially if the package of the invention contains a wound dressing composition in dry from, especially in form of a xerogel, sponge of film. Such packages are especially preferred embodiments of the invention.

In another embodiment of the present invention the wound dressing compositions are located on an inert support, preferably selected from adhesive strip, adhesive wrap, bandage, gauze bandage or compress system. Thus, the present invention also relates to wound dressing systems comprising an inert support and a wound dressing composition of the invention. Such wound dressing systems can also be packaged in a microorganism-impermeable container or foil and thus represent wound dressing packages with high patient compliance.

The wound dressing compositions, systems and packages of the present invention may be used for the treatment of wounds, especially badly healing wounds like chronic wounds, in particular for the treatment of diabetic, venous, decubitus or neuropathic ulcers or infected wounds.

Moreover, the present compositions may also be used for cosmetic or medical treatment of skin diseases and skin conditions or diseases of a mucosa or eye in general, especially when an active ingredient is part of the compositions and the compositions therefore serve as topical delivery system for the active ingredient. Dependent on the therapeutic or cosmetic aspect of the applied active ingredients the compositions suitable for cosmetic and/or medical use of the invention can be used, for example for treating and/or preventing ophthalmologic, nasal diseases, wounds or skin disorders, e.g. selected from psoriasis, dermatoses, eczema, urticaria, lupus erythematous, vitiligo, pigmentation disorders, wrinkling, aged skin, ichthyoses, hyperkeratoses, contact dermatitis, hand eczema or atopic dermatitis. It may also be used, depending on the active ingredient, for transdermal delivery of active ingredients.

The invention therefore relates to a wound dressing composition according to the present invention comprising HEC and Gellan gum, wherein the composition further comprises one or more active ingredients. In another embodiment the invention relates to a a wound dressing composition according to the present invention comprising HEC and Gellan gum, wherein the composition further comprises one or more active ingredients for use as a medicament. Also, the invention relates to the use of a wound dressing composition according to the present invention comprising HEC and Gellan gum for the preparation of a medicament for the treatment and/or prevention of ophthalmologic, nasal diseases, wounds and skin disorders.

Thus the present invention also relates to wound dressing composition of the present invention, wherein the composition further comprises one or more active ingredients which are active in treatment and/or prevention of ophthalmologic, nasal diseases or skin disorders, e.g. selected from psoriasis, dermatoses, eczema, urticaria, lupus erythematous, vitiligo, pigmentation disorders, wrinkling, aged skin, ichthyoses, hyperkeratoses, contact dermatitis, hand eczema or atopic dermatitis.

Such active ingredients comprise steroids, like hydrocortisone and betamethasone, Calcineurin inhibitors like tacrolimus, Pimecrolimus and Cyclosporin A, clobetasol, vitamin A derivatives like retinoic acid and esters and amides thereof, Tazarotene, vitamin D derivatives like calcitriol and calcipotriol, to enumerate a few. Furthermore, the invention thus relates to the use of a wound dressing composition of the present invention comprising at least one active ingredient for the preparation of the medicament for the treatment of mucosal diseases, ophthalmologic diseases, wounds and skin diseases.

The wound healing compositions can be used directly for the treatment of wounds, without any further active ingredient due to the beneficial effects of the components HEC and gellan gum on wound healing alone as well as their optimal rheologic and moist properties when mixed to homogeneity. The invention therefore relates to a wound dressing composition of the present invention for use as a medicament.

In a preferred embodiment, the invention thus relates to the use of a wound dressing composition of the present invention for the preparation of the medicament for the treatment of wounds, especially badly healing wounds like chronic wounds, in particular for the treatment of diabetic, venous or neuropathic ulcers or infected wounds.

If body fluid, for example wound secretion, nose secret or eye drops, exists at the place of use the wound dressing composition suitable for cosmetic and/or medical use can be applied directly, independent of whether it is in a "wet" state, especially as a hydrogel or in a dry storage form, especially as a xerogel or film.

Dry wound dressing compositions of the present invention can alternatively be reconstituted with water or other adequate solutions before application. This is necessary especially when compositions are to be applied to dry skin surfaces or if specific, especially rheologic, characteristics of the rehydrated hydrogel are needed. Thus, depending on the viscosity to be achieved, the hydration medium can be selected as needed.

In one embodiment the present invention relates to a method for treating wounds comprising the steps of:
1) Providing a dry wound dressing composition comprising a mixture of hydroxyethyl cellulose and gellan gum, and where necessary, one or more active ingredients, in an amount that the mixture will form a topical gel upon hydration of the mixture
2) Hydration of the mixture in a sufficient amount of water or liquid with low electrolyte content to form a free-flowing gel
3) applying a therapeutically effective amount of the gel topically to a wound.
4) optionally covering the wound for example with a plaster, compress or bandage

In another embodiment the present invention relates to a method for treating wounds comprising the steps of:
1) Providing a dry wound dressing composition comprising a mixture of hydroxyethyl cellulose and gellan gum, and where necessary, one or more active ingredients, in an amount that the mixture will form a topical gel upon hydration of the mixture
2) Hydration of the mixture in a sufficient amount of liquid with a sufficiently high electrolyte content to form a immobilized gel.
3) applying a therapeutically effective amount of the gel topically on top of the free-flowing gel from the preceding method to the wound; in this case step 4 of the preceding method, i.e. covering the wound for example with a plaster, compress or bandage, may be performed after step 3 of the present method.

In another embodiment the present invention relates to a method for treating wounds comprising the steps of:
1) Providing a dry wound dressing composition comprising a mixture of hydroxyethyl cellulose and gellan gum, and where necessary, one or more active ingredients, in an amount that the mixture will form a topical gel upon hydration of the mixture
2) Hydration of the mixture in a sufficient amount of liquid with a sufficiently high electrolyte content to form a immobilized gel.
3) applying a therapeutically effective amount of the gel topically to a wound.
4) optionally covering the wound for example with a plaster, compress or bandage

As described above, the wound dressing compositions comprising a gellan gum and HEC, especially in form of a hydrogel, are per se beneficial for wound wound healing. However, active substances beneficial for wound healing, in the following called wound healing therapeutic substances, may be added in order to further improve the beneficial effect of the compositions on the wound healing process.

Surprisingly it has been found that the HEC/gellan gum wound dressing compositions of the invention have also a excellent ability to stabilize and deliver proteins in a controlled way, in particular when the protein is a protease inhibitor, most particularly when the protease inhibitor is alpha-1-antichymotrypsin. It was found that the wound dressing compositions of the present invention are suitable for stabilizing ACT in a storage form allowing easy storage, and which release ACT at a controlled rate (see example 7).

Thus the present invention also relates to wound dressing compositions of the present invention, wherein the compositions further comprise one or more wound healing therapeutic substances.

Such wound healing therapeutic substance may be present in a concentration of about up to about 10% by weight, preferably from about 0,001 to about 5% by weight, typically from about 0,1 to about 2% by weight of one or more wound healing therapeutic agents, such as non-steroidal anti inflammatory drugs e.g. acetaminophen, steroids, like hydrocortisone or betamethosone, local anaesthetics, antimicrobial agents, growth factors ((e.g. fibroblast growth factors or platelet derived growth factor), or protease inhibitors, in particular alpha-1-antichymotrypsin (ACT) or alpha-1-antitrypsin (AAT). The antimicrobial agent may, for example, comprise an antiseptic, an antibiotic, or mixtures thereof. Preferred antibiotics include cephalosporins (cephalexin, cefoxytin, and others), penicillins (amoxycillin, ampicillin, phenoxymethylpenicillin, and others), tetracyclines (minocycline, doxycycline, and others), aminoglycosides (gentamicin, neomycin, and others), antifungals (isoconazole, clotrimazole, amphotericin, and others), sulphadiazine, chloramphenicol, erythromycin, vancomycin, trimethoprim, and others. Preferred antiseptics include silver, including colloidal silver, silver salts including one or more silver salts of one or more of the anionic polymers making up the material, silver sulfadiazine, chlorhexidine, povidone iodine, triclosan, sucralfate, quarternary ammonium salts and mixtures thereof. The concentrations refer to the concentration in the wet, hydrogel state of the wound dressing compositions of the invention.

In a preferred embodiment the wound healing therapeutic substance is a protein. In a more preferred embodiment, the wound healing therapeutic substance is a protease inhibitor belonging to the Serpin family. Preferred serpins are ACT, alpha-1-antitrypsin, antithrombin III, alpha-2-antiplasmin, C1 Inhibitor (C1INH), pancreatic trypsin inhibitor, plasminogen activator inhibitor-1 (PAI-1), Plasminogen activator inhibitor type-2 (PAI-2), Heparin Cofactor II, active protein C inhibitor, PN-1, Maspin, SERPINB12, Protease inhibitor 14, SERPINB3 and -4, SERPINB 1. In an even more preferred embodiment, the protease is selected from ACT and alpha-1-antitrypsin. In the most preferred embodiment of the invention the wound healing therapeutic substance is ACT.

In another preferred embodiment, a wound healing composition in a wet, hydrogel state comprising a gellan gum, HEC and a wound healing therapeutic substance, particularly ACT, contains the wound healing therapeutic substance in a concentration of about ACT is between 10 µg/ml and 10 mg/ml in the wet, hydrogel state, preferably between 100 µg/ml and 10 mg/ml.

In another preferred embodiment, a dry wound healing composition comprising a gellan gum, HEC and a wound healing therapeutic substance, particularly ACT, contains the wound healing therapeutic substance in a concentration of about between 0,1 µg/cm² and 1000 µg/cm² of wound dressing surface.

In another aspect, the present invention makes available a method of treatment of a chronic wound in a mammal, such as a neuropathic ulcer decubitus ulcer, a venous ulcer or a diabetic ulcer or an infected wound. The method comprises applying a wound dressing according to the invention comprising a wound healing therapeutic substance to the wound.

Typically, the wound dressing according to the invention is applied to the chronic wound for a period of at least 1 hour, preferably at least 24 hours, more preferably at least 48 hours, and most preferably at least 72 hours.
The treatment may be extended for several days, weeks or months, with dressing changes as appropriate, if necessary for chronic wounds.

The production of sterile wound healing compositions can be performed as follows comprising the steps of:
HEC and gellan gum as dry powders are levigated in a mortar and thereby mixed. The mixture is dispersed in a suitable solvent, preferably an aqueous solution and is homogenised by stirring. The dispersion is then autoclaved, whereby sterility and the gelling notably of the gellan gum component, which is temperature dependent, is achieved at the same time. The preparation is poured at a temperature between 70°C to 90°C into a suitable dish or may get cast out with a scraper on a suitable base. Solvent is removed from the dispersion to leave a solid material comprising a homogeneous mixture of HEC and gellan gum.

The optional, additional components in the materials according to the present invention are preferably included in the dispersion prior to autoclavation. For materials that do not tolerate this procedure and/or for temperature-sensitive substances like proteins, comprising proteinogenic protease inhibitors, in particular alpha-1-antichymotrypsin, an additional working step may be added:
after the removal of solvent the solid is again hydrated by the addition of a suitable solution of the protein or other sensitive substance with suitable excipients. Then an additional step of removing the solvent is performed.

The solvent can be removed from the dispersion by evaporation, for example by evaporation from the dispersion in a tray resulting in a dry film. In other embodiments the solvent, preferably water, is removed by freeze-drying (lyophilising) or solvent-drying to obtain the material in the form of a xerogel or sponge. Preferably, the method of lyophilisation is carried out including an annealing step before primary drying, see example

"Xerogel" according to the present invention is to be understood as porous, sponge-like matrix obtainable from a hydrogel e.g. by freeze-drying comprising at least one gelating substance wherein the matrix has the potential to swell and form hydrogels when in contact with aqueous solutions.

"Film" according to the present invention is to be understood as polymer-based foil of flat-shaped form of uniform thickness and consistency obtainable from a hydrogel by drying, e.g. evaporative drying or by casting from organic solutions. The matrix has the potential to swell and form hydrogels when in contact with aqueous solutions.

"Dry" according to the present invention is to be understood as containing a very low content of water, preferably less than 5% (w/w) moisture, more preferably less than 2% (w/w) moisture, especially preferably less than 1% (w/w) moisture. Moisture can be determined by coulometric Karl-Fischer titration, for example using KF 373 (Metrohm GmbH & Co, Filderstadt, Germany).

"Active ingredient" is to be understood as any substance which causes a biological effect, either directly or when released from its pro-drug form in vivo and which is thus beneficial for the medical treatment or prevention of diseases and /or disorders or for cosmetic treatment of conditions of the body.

"wound" is to e understood as any injury of the skin resulting in a partial or complete destruction of the skin barrier function by partial or complete local destruction of the skin integrity. Such wounds may result from mechanical impact as slash wounds or stab wounds, or maceration and/or ulceration as observed in decubitus ulcers or diabetic ulcers, or burn wounds resulting from high temperature or chemicals. Preferred wounds according to the present invention are chronic wounds which heal slowly or badly or not all under standard treatment, especially diabetic ulcer, venous ulcers, neuropathic ulcers and decubitus ulcers or infected wounds.

"alphal-antichymotrypsin" or "ACT" according to the present invention is to be understood as an alphal-antichymotrypsin protein showing at least approximately 70%, in particular at least approx. 80%, especially at least approx. 90%, more preferred at least 95%, even more preferred at least 98% sequence identity to human wildtype alphal-antichymotrypsin and having at least 1 %, more preferably at least 10%, even more preferably at least 50% inhibitory activity versus Cathepsin G compared to mature wildtype alphal-antichymotrypsin isolated from human blood serum. The inhibitory action can be determined by assays well known in the art. Such assays are for example described in Heidtmann et al., 1990, Clin Chem 36: 2077-2081 and in Example 7. An ACT protein according to the present invention may be isolated from a mammalian organism, preferably from human serum or may be produced recombinantly, for example by use of viral, bacterial, fungal or mammalian expression systems. Such ACT may be glycosylated, partially glycosylated or unglycosylated. In a preferred embodiment, ACT is mature human wildtype ACT. In another embodiment, the ACT lacks all or part of the N-terminal signal sequence, more particularly lacks the signal sequence.

"Sequence identity" is understood as degree of identity (% identity) of two sequences, that in the case of polypeptides can be determined by means of for example BlastP 2.0.1 and in the case of nucleic acids by means of for example BLASTN 2.014, wherein the filter is set off and BLOSUM is 62 (Altschul et al., 1997, Nucleic Acids Res., 25:3389-3402). In humans, one ACT gene encoding ACT is known, with polymorphism having been described, particularly in the signal peptide sequence (Rubin, 1989, database entry). The sequence of human wildtype ACT polypeptide sequence with signal peptide is shown in SEQ ID No. 1.

The sequence of a mature human ACT polypeptide sequence without signal peptide is shown in SEQ ID No. 2 (Lindmark et al., Biochim. Biophys. Acta 997:90-95(1989)). The sequence of another, in vivo predominant, mature ACT polypeptide is shown in SEQ ID No. 3. Both mature ACT polypeptides are obtained by cleavage of the signal peptide. ACT polypeptides which exhibit ACT activity comparable to ACT polypeptides according to SEQ ID No. 2 and 3 are polypeptides according to SEQ ID No. 4 and SEQ ID No. 5 which can be e.g. obtained by recombinant expression in yeast and which are characterized by an N-terminal Methionine followed by the sequence of the mature ACT polypeptides according to SEQ ID No. 7 and SEQ ID No. 10. Such N-terminal extensions, for example a Methionine extension which may be introduced in order to facilitate expression and/or purification do not affect ACT activity.

The polypeptides according to any of SEQ ID No. 1 to 5 and functional variants thereof are preferred ACT polypeptides according to the invention. Particularly preferred is the use of mature ACT polypeptides lacking the signal peptide according to SEQ ID No. 2 to 3 and functional variants thereof, especially functional variants according to SEQ ID No 4 to 5.

The term "functional variants" is to be understood as meaning variants of the ACT polypeptides, which can be used in accordance with the invention, which variants possess protease inhibitor specificity with regard to cathepsin G which is similar to that of the mature wildtype ACT polypeptide. For example, functional variants of ACT polypeptides according to SEQ ID No. 1 to 5 possess at least approximately 70%, in particular at least approx. 80%, especially at least approx. 90%, more preferred at least 95%, even more preferred at least 98% sequence identity with one of the sequences SEQ ID No. 1, SEQ ID No.: 3, SEQ ID No.: 4, or SEQ ID No. 5 and have the Cathepsin G protease inhibitory activity similar to that of the mature wildtype ACT polypeptide which can be determined as described in Example 7.

"Similar inhibitory activity" is to be understood as activity which is at least 1%, preferably at least 10%, even more preferably at least 50% of the activity of a mature human wildtype ACT polypeptide.

Functional variants of the polypeptides can also be parts of the polypeptides used in accordance with the invention which, when compared with the wildtype ACT polypeptides, do exhibit similar protease inhibitor activity. For example, the first amino acid, i.e. methionine, can be missing in a polypeptide without there being any significant change in the function of the polypeptide. Also, as mentioned above for SEQ ID No. 4 and 5, an N-terminal Methionine may be added to an ACT polypeptide or functional variants thereof, e.g. a methionine may be added to the N-terminus of a mature ACT polypeptide without there being any significant change in the function of the polypeptide. N- and/or C-terminal and/or internal deletions of the polypeptide in the range of approx. 1-60, preferably of approx. 1-30, in particular of approx. 1-15, especially of approx. 1-5, amino acids are also included provided the protease inhibitor specificity remains essentially unaltered as compared with that of the respective wildtype polypeptide. Particular preference is given to deletions which affect the signal peptide, or parts thereof, at the N terminus of an ACT polypeptide; e.g. it was shown that an ACT polypeptide having the last 4 amino acids of the signal peptides followed by the sequence of the mature ACT polypeptide according to SEQ ID No. 3 retains full ACT activity (US 5,367,064). Especially preferred are ACT polypeptides which are characterized in that all or part of the signal peptide of an ACT polypeptide according to SEQ ID No. 1 is missing, especially ACT polypeptides according to SEQ ID No. 2 and SEQ ID No. 3. ACT polypeptides having an additional N-terminal Methionine are also preferred ACT polypeptides according to the invention, especially ACT polypeptides according to SEQ ID No. 4 and SEQ ID No. 5.

### Figure Legend:

Figure 1 shows the results of example 7.
Figure 2 shows a xerogel pad after freeze drying without annealing step
Picture 3 shows a xerogel pad after freeze drying with annealing step

### Examples

Specific embodiments of the present invention will now be described further, by way of example.

### Example 1: Preparation of xerogels comprising HEC, gellan gum and ACT in vials

(1) 2g Hydroxyethyl cellulose (Natrosol® 250 HHX, purchased from Dow chemicals) and 1g gellan gum (Kelcogel F, purchased from Kelko) as dry powders are levigated in a mortar and so intimately mixed .
(2) The mixed powder is dispersed in 97g of demineralised water with a propeller mixer at high stirring speed. The dispersion is stirred several minutes until it appears homogeneous.
(3) The semisolid mass is filled into infusion bottles and which is then sealed. In an autoclave a standard sterilisation program is carried out (121°C, 15 minutes, 2 bar).
   With this step sterility and the gelling notably of the gellan gum component (which is temperature dependent) is achieved at the same time.
(4) Under sterile conditions the preparation is poured at a temperature between 70°C to 90°C into a petri dish to a height of 4 mm. Under these conditions the mass is freely flowing and forms a cylindrical shape with uniform surface and thickness in the dish .
(5) Water is removed from the dispersion by drying at 25°C under a flow of warm (40°C) nitrogen gas. At the end of drying a uniform dry film is left in the dish which consists of homogeneously mixed HEC and gellan gum.
(6) For ACT loading simply by hydration, a first solution without the protein containing a 10 mM potassium phosphate buffer, 0,1 % of Poloxamer 188 (BASF AG, Ludwigshafen, Germany) and 0,05% of Kollidon 17PF (BASF AG, Ludwigshafen, Germany) is prepared and sterilized by autoclavation. Under aseptic conditions the alpha-1-antichymotrypsin is dissolved and the solution is again filtered through a 0,22 µm unit to provide sterility for the whole solution.
(7) The film is cut in pieces that exactly cover the bottom of glass vials. The film is placed in the vial and is hydrated with the ACT containing solution of step (6) to a filling height of 4 mm In a 2R vial the necessary amount is 0.4ml.. The mixture is allowed to swell for at least 24 hours to again form a homogeneous hydrogel. By this form of drug loading almost no shear stress (which is a critical part in protein handling) is applied to the drug substance.
(8) The vial with the gel inside is now placed into a freeze dryer at 20°C. The lyophilisation is carried out following the program in example 9:

The dry pad has a homogeneous appearance and its mechanical properties are very suitable for proper handling by both patients and personnel. It swells within minutes to form a hydrogel when put in contact with an aqueous solution. Once rehydrated, the gel very soft and feels comfortable when put onto a wound. It also provides intimate contact with the wound ground to ensure the proper release of the active ingredient load.

### Example 2: Preparation of xerogels comprising HEC, gellan gum and ACT in sheets of 2 mm height

(1) The preparation of the xerogel in sheets follows the same procedure as the preparation of the xerogel in vials from step ACT containing solution of step (6) of example 1 is poured onto the placebo film in the dish to a height of 4mm and is swollen for at least 24 h.
(2) The hydrated gel is heated to 40°C to achieve good flowability and is filled into a scraper. The gel now is cast out on a glass base to produce a wet film with a height of 2mm.

Step (9) is carried out like step (8) above in example 1.

The matrix is obtained in sheets and can be cut into pieces suitable for further packaging. This simplifies production processes.

### Example 3: Preparation of films comprising HEC, gellan gum and ACT in vials

The preparation of the film follows the same procedure as the preparation of the xerogel from step (1) to (5).
(6) Then for ACT loading simply by hydration a first solution without the protein containing a 5 mM potassium phosphate buffer, 0,1 % of Poloxamer 188; 1,0% PEG 400, and 1,5% of Kollidon 17PF is made and gets sterilized by autoclavation. Under aseptic conditions the alpha-1-antichymotrypsin is dissolved and the solution is again filtered through a 0,22 µm unit to provide sterility for the whole solution.
(7) The film is cut in pieces that exactly cover the bottom of glass vials. The film is placed in the vial and is hydrated with the ACT containing solution to a filling height of 4 mm. The mixture is allowed to swell for at least 24 hours to form a homogeneous hydrogel again. By this form of drug loading almost no shear stress which is a critical part in protein handling is applied to the drug substance.
(8) Water is removed from the dispersion by drying at 25°C under a flow of warm (40°C) nitrogen gas.

The obtained film is very soft and elastic but provides the necessary robustness for good handling qualities. It swells when hydrated to form a clear gel within about 45 minutes. Once rehydrated the gel on the wound is very soft and comfortable. It also provides intimate contact with the wound ground to ensure the proper release of the active ingredient load.

### Example 4: Preparation of films comprising HEC, gellan gum and ACT in sheets

The preparation of the film follows the same procedure as the preparation of the xerogel from step (1) to (5) in example 1.
(6) For ACT loading simply by hydration a first solution without the protein containing a 5 mM potassium phosphate buffer, 0,1 % of Poloxamer 188,0, 1,0 % PEG 400, and 1,5 % of Kollidon 17PF is prepared and sterilized by autoclavation. Under aseptic conditions the alpha-1-antichymotrypsin is dissolved and the solution is again filtered through a 0.22 µm unit to provide sterility for the whole solution.
(7) The ACT containing solution is poured onto the placebo film in the dish to a height of 4 mm and is swollen for at least 24 h.
(8) The hydrated gel is heated to 40°C to achieve good flowability and is filled into a scraper. The gel now is cast out on a glass base to produce a wet film with a height of 2 mm.
(9) Water is removed from the dispersion by drying at 25°C under a flow of warm (40°C) nitrogen gas.

The matrix is obtained in sheets and can be cut into pieces suitable for further packaging. This simplifies production processes.

### Example 5: Preparation of HEC/Gellan gum xerogels without active ingredient

The xerogels are produced like in example 2 but without the steps (4) to (7). The product has the same properties than example 2. It can for example be used as a wound dressing.

### Example 6: Preparation of HEC/Gellan gum films without active ingredient

The films a produced like in example 4 but without the steps (4) to (7). The product has the same properties than example 4. It can for example be used as a wound dressing.

### Example 7: Release of ACT from wound dressing compositions comprsing HEC, gellan gum and ACT and determination of stability

In order to determine the suitability of ACT-containing wound dressing compositions of the invention, release tests were carried out in vitro. Model chambers described in Loth,H. & Holla-Benninger,A. (Studies on the drug release from ointments. Part 1. Development of an in vitro release model. *Pharmazeutische Industrie* 40, 256-261 (1978)) were modified in that way that the donor chamber has only 1.25 ml volume and is cylindrical in shape with a diameter of 2 cm. The dividing membrane between donor and acceptor is a cellulose acetate filter membrane of 0.45 µm pore size. Therefore, it does not simulate a physiological barrier like skin, but it separates the chambers sterically and thereby mimicks the situation in a wound that also lacks of a skin barrier. The pore size is chosen in a way that the membrane provides a barrier for the large gelling agent molecules, but does not remarkably influence the diffusion of ACT. The acceptor consists of 10 mM potassium phosphate buffer pH 7.2. The reconstitution of the dried forms is done with phosphate-buffered saline (PBS).
In order to determine the ACT activity, an activity assay based on Cathepsin G binding was performed. 96-well plates were coated with BSA and subsequently with Cathepsin G. After washing, ACT probes were added and incubated for 30 min at 37°C. After washing with PBS-T 3 times, a rabbit anti human ACT antibody was added to the wells and was incubated for 30 min at 37°C. After washing three times with PBS-T buffer, a goat anti rabbit IgG antibody conjugated with horseradish peroxidase was added and was again incubated for 30 min at 37°C. The wells were washed three times with PBS-T buffer. Subsequently OPD (1,2-Diaminobenzene) substrate solution was prepared according to the manufacturer's protocol (Sigma), added to the wells and incubated at room temperature in the dark. After 10 minutes the reaction was stopped by adding 100 µl 0,5 M H2SO4 per well. Immediately after stopping the reaction the absorption at 490 nm was determined.

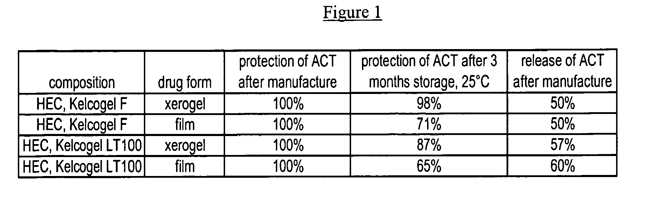

The percentage of the bioactive fraction of the total ACT amount in each sample was determined and expressed in Figure 1 as percentage in comparison with untreated samples of ACT. The release maximum is determined 5 days after start of diffusion when an equilibrium is reached.

Before drying all gel formulations contain 0.1% Poloxamer 188®, 10 mM arginine, 10 mM phosphate buffer. The HEC/Gellan gum (Kelcogel) mixtures contain 2% HEC and 1 % gellan gum in the wet state. HEC gels contain 2.5% of hydroxyethyl cellulose without gellan gum (Figure 1). HEC is hydroxyethyl cellulose (Natrosol® 250 HHXpharm, Dow Chemicals), two different types of Gellan gum were tested: Kelcogel F® (Kelco) as a deacetylated Gellan Gum, and Kelcogel LT100® (Kelco), as an acetylated Gellan gum.

It was observed that especially dry storage forms of homogeneous mixtures of deacetylated gellan gum and HEC result in wound dressing compositions which excellently stabilize and release ACT after manufacture. Especially xerogels comprising HEC and deacetylated Gellan gum provide both excellent stabilization and release of ACT. Moreover, the rheological characterisitics of the wound dressing compositions are excellent. Thus, the compositions of the invention comprising ACT are especially suitable for use in wound healing.

### Example 9: Freeze drying of hydrogels to form xerogels

The freeze drying operation is carried out in a Christ® Lab scale freeze dryer. The program is as follows:
Start 20 °C 1000 mbar
1 h -15 °C 1000 mbar
1,5 h -15 °C 1000 mbar
1 h -1 °C 1000 mbar
2,5 h -1 °C 1000 mbar
2 h -40 °C 1000 mbar
1,5 h -40 °C 1000 mbar
0,5 h -25 °C 0,5 mbar
24 h -25 °C 0,5 mbar
0,1 h -25°C 0,001 mbar
5 h 20°C 0,001 mbar
12 h 20°C 0,001 mbar

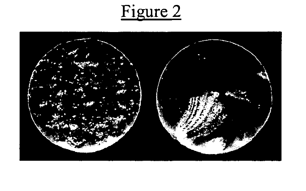

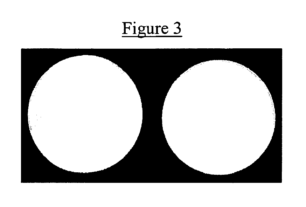

Surprisingly, we found out that the annealing step that again heats up to -1°C after 2.5 h and the low freeze speed of 0.65 °C/min after 5 h before primary drying are essential for the homogeneous appearance of the xerogel pad (Figure 2 versus Figure 3). This is very important for patient and medical personnel compliance.

## Claims

1. Wound dressing composition comprising a cellulose ether and a gellan gum.

2. Wound dressing composition according to claim 1 comprising a hydroxyethyl cellulose and a gellan gum.

3. Wound dressing composition according to claim 1 or 2, wherein the cellulose ether and the gellan gum are homogeneously mixed.

4. Wound dressing composition according to any of claims 1 to 3, wherein the gellan gum is deacetylated gellan gum.

5. Wound dressing according to any of the foregoing claims, **characterized in that** the composition is dry.

6. Wound dressing composition according to claim 5, wherein the composition is a film.

7. Wound dressing composition according to claim 5, wherein the composition is a xerogel or sponge.

8. Wound dressing composition according to any of claims 1 to 4, **characterized in that** the composition is a hydrogel.

9. Wound dressing composition according to claim 8, obtainable by rehydration of a dry wound dressing composition according to claims 5 to 7.

10. Wound dressing composition according to any of the foregoing claims **characterized in that** the composition contains additional excipients.

11. Wound dressing composition according to any of the foregoing claims, wherein the hydroxyethyl cellulose and gellan gum together make up at least 25% by weight of the material on a dry weight basis, preferably at least 50% by weight of the material on a dry weight basis.

12. Wound dressing composition according to any of the foregoing claims, wherein the weight ratio of hydroxyethyl cellulose to gellan gum is in the range of 1:5 to 50:1 , preferably 1:1 to 10:1.

13. Wound dressing package comprising a sterile wound dressing composition according to any of the foregoing claims, packaged in a microorganism-impermeable container.

14. Wound dressing package according to claim 13, wherein the sterile wound dressing composition is a composition according to any of claims 5 to 7.

15. Wound dressing package comprising a sterile wound dressing composition according to any of the claims 1 to 12 and an inert support, preferably selected from adhesive strip, adhesive wrap, bandage, gauze bandage and compress system.

16. Wound dressing composition or wound dressing package according to any of the foregoing claims for use as a medicament.

17. Use of a wound dressing composition or wound dressing package according to any of the claims 1 to 15 for the preparation of the medicament for the treatment of wounds, especially chronic wounds.

18. Use according to claim 17 wherein the said chronic wound is selected from the group consisting of venous ulcers, decubitus ulcers, neuropathic ulcers, diabetic ulcers and infected wounds.

19. Wound dressing composition according to any of the claims 1 to 12, wherein the composition further comprises one or more active ingredients.

20. Use of a wound dressing composition according to claim 19 for the preparation of a medicament for the treatment and/or prevention of ophthalmologic, nasal diseases, wounds and skin disorders.

21. Wound dressing composition comprising a cellulose ether and a gellan gum and one or more wound healing therapeutic substances.

22. Wound dressing composition according to claim 21, comprising a hydroxyethyl cellulose and a gellan gum and one or more wound healing therapeutic substances.

23. Wound dressing composition according to claim 21 or 22, wherein the cellulose ether and the gellan gum are homogeneously mixed.

24. Wound dressing composition according to any of claims 21 to 23, wherein the gellan gum is deacetylated gellan gum.

25. Wound dressing according to any of the claims 21 to 24, **characterized** that the composition is dry.

26. Wound dressing composition according to claim 25, wherein the composition is a film.

27. Wound dressing composition according to claim 25, wherein the composition is a xerogel or sponge.

28. Wound dressing composition according to any of claims 21 to 24, **characterized in that** the composition is a hydrogel.

29. Wound dressing composition according to claim 28, obtainable by rehydration of a dry wound dressing composition according to claims 25 to 27.

30. Wound dressing composition according to any of the claims 21 to 29 **characterized in that** the composition contains additional excipients.

31. Wound dressing composition according to any of the claims 21-30, wherein the hydroxyethyl cellulose and gellan gum together make up at least 25% by weight of the material on a dry weight basis, preferably at least 50% by weight of the material on a dry weight basis.

32. Wound dressing composition according to any of the claims 21-32, wherein the weight ratio of hydroxyethyl cellulose to gellan gum is in the range of 1:5 to 50:1, preferably 1:1 to 10:1.

33. Wound dressing package comprising a sterile wound dressing composition according to any of the claims 21 to 29, packaged in a microorganism-impermeable container.

34. Wound dressing package according to claim 33, wherein the sterile wound dressing composition is a composition according to any of claims 25 to 27.

35. Wound dressing package comprising a sterile wound dressing composition according to any of the claims 21-32 and an inert support, preferably selected from adhesive strip, adhesive wrap, bandage, gauze bandage and compress system.

36. Wound dressing composition or wound dressing package according to any of the claims 21-35 for use as a medicament.

37. Use of a wound dressing composition or wound dressing package to any of the claims 21-35 for the preparation of the medicament for the treatment of wounds, especially chronic wounds.

38. Use according to claim 37 wherein the said chronic wound is selected from the group consisting of venous ulcers, decubitus ulcers, neuropathic ulcers, diabetic ulcers and infected wounds.

39. Wound dressing composition according to any of the claims 21 to 32, wherein the composition further comprises one or more active ingredients.

40. Use of a wound dressing composition according to claim 39 for the preparation of a medicament for the treatment and/or prevention of ophthalmologic, nasal diseases, wounds and skin disorders.

41. Wound dressing composition or wound dressing package according to any of claims 21 to 36, **characterized in that** the wound healing therapeutic substance is a protein.

42. Wound dressing composition or wound dressing package according to any of claims 21 to 36 and 41, **characterized in that** the wound healing therapeutic substance is a protease inhibitor.

43. Wound dressing composition or wound dressing package according to claim 42, **characterized in that** the wound healing therapeutic substance is a serpin, preferably selected from the group consisting of alpha-1-antichymotrypsin, alpha-1-antitrypsin, antithrombin III, alpha-2-antiplasmin, C1 Inhibitor (C1INH), pancreatic trypsin inhibitor, plasminogen activator inhibitor-1 (PAI-1), Plasminogen activator inhibitor type-2 (PAI-2), Heparin Cofactor II, active protein C inhibitor, PN-1, Maspin, SERPINB12, Protease inhibitor 14, SERPINB3 and-4, SERPINB1.

44. Wound dressing composition or wound dressing package according to claim 43, **characterized in that** the wound healing therapeutic substance is alpha-1-antichymotrypsin.

45. Wound dressing composition according to claim 44, **characterized in that** alpha-1-antichymotrypsin is present in a concentration of about between 1 µg/ml and 10 mg/ml in the wet state, preferably between 100 µg/ml and 10 mg/ml in the wet state.
